# EUROPÄISCHE PATENTSCHRIFT

(11) **EP 1 131 101 B1**
(45) Veröffentlichungstag und Bekanntmachung des Hinweises auf die Patenterteilung: **07.01.2004**
(21) Anmeldenummer: 99972546.8
(22) Anmeldetag: 28.10.1999
(51) Int. Cl.: A61K 41/00, A61K 31/35, A61P 17/06

(54) **KHELLIN-ZUBEREITUNG UND DEREN VERWENDUNG ZUR TOPISCHEN THERAPIE**
KHELLIN PREPARATION AND THE USE THEREOF FOR TOPICAL THERAPY
PREPARATION DE KHELLINE ET SON UTILISATION POUR DES TRAITEMENTS A ACTION LOCALE

(30) Priorität: 19.11.1998 DE 19853425
(43) Veröffentlichungstag der Anmeldung: 12.09.2001
(73) Patentinhaber: Röcken, Martin, 80337 München (DE); Messer, Gerald, 80337 München (DE)
(72) Erfinder: Röcken, Martin, 80337 München (DE); Walchner-Bonjean, Monika, 80337 München (DE); Messer, Gerald, 80337 München (DE)
(74) Vertreter: Grättinger & Partner (GbR)
(86) Internationale Anmeldenummer: PCT/EP1999/008175
(87) Internationale Veröffentlichungsnummer: WO 2000/030682

(56) Entgegenhaltungen:
- EP-A- 0 680 761
- WO-A-89/06702
- WO-A-96/08965
- WO-A-97/05127
- DE-U- 29 820 719
- DATABASE EMBASE [Online] ELSEVIER SCIENCE PUBLISHERS, AMSTERDAM, NL PROCACCINI E.M. ET AL: "[Evaluation of topical khellin photochemotherapy for vitiligo]. VALUTAZIONE DELL'EFFICACIA DELLA FOTOCHEMIOTERAPIA DELLA VITILIGINE CON KELLINA TOPICA." retrieved from STN Database accession no. 94031127 XP002129871 & ANNALI ITALIANI DI DERMATOLOGIA CLINICA E SPERIMENTALE, (1993) 47/4 (275-278). ,
- DATABASE CHEMABS [Online] CHEMICAL ABSTRACTS SERVICE, COLUMBUS, OHIO, US ORECCHIA, G. ET AL: "Topical photochemotherapy of vitiligo with a new khellin formulation: preliminary clinical results" retrieved from STN Database accession no. 130:20529 XP002129872 & J. DERMATOL. TREAT. (1998), 9(2), 65-69 ,
- DATABASE CHEMABS [Online] CHEMICAL ABSTRACTS SERVICE, COLUMBUS, OHIO, US GIORDANO, FERDINANDO ET AL: "Preformulation studies and formulation strategies on khellin, an old/new antivitiligo agent. Interaction with cyclodextrins, solubility in mixed solvent systems, and permeation through artificial membranes" retrieved from STN Database accession no. 123:208591 XP002129873 & EUR. J. PHARM. BIOPHARM. (1994), 40(4), 232-6 ,

## Beschreibung

Die Erfindung betrifft die Verwendung von Khellin zur Herstellung eines Arzneimittels zur phototherapeutischen Behandlung der Psoriasis, ihrer Subtypen, Sonderformen sowie von Ekzemen. Sie betrifft ferner die Verwendung von Khellin zur Herstellung einer neuen äußerlich anwendbaren, für die Applikation auf der Haut geeigneten Khellin-Zubereitung zur Behandlung der Schuppenflechte (Psoriasis).

Die Schuppenflechte gehört zu den häufigsten Hauterkrankungen mit einer schwerwiegenden psychosozialen Belastung für die betroffenen Patienten. Das Krankheitsbild ist durch scharf begrenzte erythematöse, mit silbrigweißen Schuppen bedeckte, zuweilen juckende Plaques verschiedener Größe und Gestalt gekennzeichnet. Unterschiedliche genetische Veranlagungen zusammen mit auslösenden Triggerfaktoren wie bakteriellen Infektionen, Streß und Medikamenten führen zu einem weiten Spektrum klinischer Manifestationen. Die Erkrankung weist in der Regel einen chronisch-rezidivierenden Verlauf auf.

Die Behandlung der Schuppenflechte zielt auf eine Remission mit einer möglichst lange anhaltenden Erscheinungsfreiheit ab. Üblicherweise wird mittelschwere bis schwere Psoriasis mit einer oralen oder örtlichen Photochemotherapie mit 8-Methoxypsoralen (8-MOP) und anschließender UVA-Bestrahlung (= PUVA-Therapie) behandelt. Wegen der begleitenden Übelkeit kann die orale Therapie oft nicht angewandt werden. Zudem ist die Behandlung von einer sehr hohen Phototoxizität begleitet und erfordert äußerste Sicherheitsvorkehrungen und eine intensive Kooperation des Patienten. Wichtige Daten legen außerdem nahe, daß die PUVA-Therapie das Auftreten von malignen Tumoren der Haut, insbesondere von spinozellulären Karzinomen provoziert. Daher wird die PUVA-Phototherapie in der Regel erst ab dem 4. - 5. Lebensjahrzehnt eingesetzt. Eine mögliche Alternative zur oralen PUVA-Therapie stellt die Bade-PUVA-Therapie mit 8-Methoxypsoralen oder Trimethylpsoralen dar. Die Karzinogenität dieser Photochemotherapie scheint günstiger zu sein als die der oralen PUVA-Therapie. Sichere Daten liegen noch nicht vor. Die Bade-PUVA-Therapien erfordern einen erheblichen Zeitaufwand, verursachen sehr große logistische Schwierigkeiten, da Badeinheit und Bestrahlungseinheit in direkter Nachbarschaft liegen müssen und sind therapeutisch schwer steuerbar, da sie mit einer sehr ausgeprägten Photosensibilität einhergehen und somit leicht phototoxische Reaktionen hervorgerufen werden können. Gegenwärtig gibt es jedoch kein zufriedenstellendes Medikament zur Behandlung der Schuppenflechte.

Exzeme entstehen entweder endogen, so das atopische Ekzem, oder durch die akute bzw. chronische Einwirkung von spezifischen (Allergen) oder unspezifischen (mechanische Reizung, Druck, Chemikalien, häufiges Reinigen der Haut) Reizen auf die Haut. Diese reagiert ihrerseits mit einem entzündlichen Infiltrat aus Lymphozyten und anderen mononukleären Zellen und möglicherweise auch verstärktem Wachstum (Proliferation). Um die Wirksamkeit der KUVA-Therapie zu analysieren, haben wir hier als Beispiel die Wirksamkeit der Behandlung bei Palmoplantarekzem untersucht.

Der Erfindung liegt daher die Aufgabe zugrunde, ein Arzneimittel zur Behandlung der Schuppenflechte bereitzustellen, das die Nachteile der bisherigen Präparate überwindet. Das Arzneimittel soll sich besonders zur phototherapeutischen Behandlung der Schuppenflechte eignen. Insbesondere soll das Arzneimittel in einer Form vorliegen, die für den Patienten akzeptabel ist und nach einer geeigneten Zeitspanne eine Remission der Symptome bewirkt. Ferner soll das Medikament sicher, leicht anzuwenden sowie nebenwirkungsarm sein. Es soll sich auch für eine Langzeittherapie eignen und schon jungen Patienten zufriedenstellend verabreicht werden können.

Überraschenderweise wurde nun gefunden, daß das Furanochromon Khellin und dessen Derivate, die zu Monoaddukten führen, insbesondere bei Formulierung als ein zur Applikation auf die Haut geeignetes Präparat, topisch zur photochemotherapeutischen Behandlung der Schuppenflechte eingesetzt werden können, ohne daß schädliche Nebenwirkungen auftraten.

Die Erfindung betrifft daher die Verwendung von Khellin zur Herstellung eines Arzneimittels zur topischen UVA-Strahlenbehandlung der Psoriasis, ihrer Subtypen und Sonderformen, einschließlich der Palmoplantar-Psoriasis, sowie zur Behandlung von Ekzemen, in einer Applikationsdosis von 0,1-15 Gew.-% bis zu einer Verdünnung auf 60-100 ng/mg Gewebe. Ferner betrifft sie die Verwendung von Khellin zur Herstellung einer streichfähigen bzw. spreitfähigen, für die Applikation auf der Haut geeigneten Khellin-Zubereitung, enthaltend Khellin, sowie eine dermatologisch verträgliche Grundlage zusammen mit rezepturbedingten Hilfs- und Zusatzstoffen, als Photochemotherapie der Psoriasis, ihrer Subtypen und Sonderformen, einschließlich der Palmoplantar-Psoriasis, sowie als Photochemotherapie von Ekzemen, in einer Applikationsdosis von 0,1-15 Gew.-% bis zu einer Verdünnung auf 60-100 ng/mg Gewebe.

4,9-Dimethoxy-7-methyl-5H-furo[3,2-g][1] benzopyran-5-on, oder Khellin, ist ein Furanochromon, das seit über 3000 Jahren für phototherapeutische Zwecke eingesetzt wird. Die Substanz wird aus den Ammeifrüchten des im Mittelmeerraum beheimateten Strauches Ammi visnaga gewonnen. Ende der 40er Jahre wurde Khellin oral als Vasodilatator eingesetzt und wurde viele Jahre als blutdrucksenkendes Medikament und Vasodilatator bei Asthma bronchiale verordnet. Es zeigten sich hierbei keine Langzeitspätfolgen. Im Gegensatz zu 8-Methoxypsoralen, einem weit verbreiteten Photosensibilisator, der zur,PUVA-Photochemotherapie eingesetzt wird, hat Khellin nur eine sehr geringe Fähigkeit, Diaddukte auszubilden.

In der Dermatologie werden Khellin-Tabletten zur oralen Khellin-UVA-Phototherapie der Vitiligo (Weißfleckenkrankheit) eingesetzt (Dermatologica, 165, 136-140, 1982). Darüber hinaus zeigen neuere Fallbeobachtungen, daß Khellin in einer Cremegrundlage in Kombination mit einer anschließenden UVA-Bestrahlung bei Alopecia areata und Vitiligo mit mäßigem Erfolg eingesetzt werden kann. Bei diesen Indikationen sollen die Ergebnisse denen der oralen PUVA-Behandlung vergleichbar sein. Ein genauer Vergleich liegt nicht vor.

Es konnte ferner gezeigt werden, daß bei oraler Verabreichung von 100 mg Khellin und anschließender Sonnenbestrahlung von 10 Patienten mit einer Plaque-Psoriasis 80% auf die Behandlung ansprachen und 6 von 8 dieser Patienten für drei Jahre symptomfrei waren (Dermatologica, 167, 109-110, 1983). Khellin wurde jedoch bisher nicht zur topischen Behandlung der Psoriasis eingesetzt. Bei einer Photochemotherapie, die unter Zuhilfenahme der Sonneneinstrahlung erfolgt, ist es nämlich sehr schwierig, den Effekt der Therapie mit Sonne von jener einer Photochemotherapie zu trennen, da die Sonnenstrahlen große Mengen an UVB-Strahlung enthalten, die selbst bereits zum Abheilen einer Psoriasis führen können.

Die Erfahrung der konventionellen Therapie der Psoriasis mit 8-MOP und anschließender UVA-Bestrahlung hat nahegelegt, daß eine phototoxische Reizung oder eine Behandlung nahe der phototoxischen Schwelle nötig ist, um eine Psoriasis möglichst erfolgreich mittels Photochemotherapie (PUVA) zu behandeln. Dies gilt aber nicht für die Behandlung der Vitiligo. Da die bei der Vitiligo beschriebene Behandlung mit Khellin und anschließender UVA-Bestrahlung nicht zu klinisch sichtbaren phototoxischen Reaktionen führt, erschien es bisher als unwahrscheinlich, daß eine topische Therapie mit Khellin und anschließender UVA-Bestrahlung sich zur Behandlung der Psoriasis eignen würde.

Es konnte nun überraschenderweise gezeigt werden, daß eine Psoriasis, einschließlich der Psoriasis palmoplantaris sowie Ekzeme erfolgreich mit Khellin behandelt werden können. Voraussetzung ist, daß Khellin oder dessen Derivate, die zu Monoaddukten führen, in einer extern auf die Haut aufzutragenden Grundlage, die hydrophobe/lipophile Substanzen aufnehmen kann, aufgetragen wird und die Haut anschließend mit UVA oder UVA1 bestrahlt wird. In Kombination mit einer anschließenden UVA-oder UVA1-Bestrahlung eignet sich diese Zubereitung mit gutem Erfolg zur topischen Verwendung in der phototherapeutischen Behandlung der Schuppenflechte. Das Präparat war gut verträglich und führte bei Verwendung mit einer anschließenden Phototherapie zu keinen negativen lokalen und systemischen Reaktionen.

Die erfindungsgemäße Khellin-Zubereitung zeigt bei Verwendung mit einer Phototherapie (Photochemotherapie) eine sehr gute Wirksamkeit bei den verschiedenen Formen der Psoriasis, der Palmoplantar-Psoriasis und den Handund Fußekzemen. Sie besitzt eine sehr gute örtliche entzündungshemmende Wirkung und führt zu einer schnellen Rückbildung der Hauterscheinungen der Psoriasis. Die Remission wurde histologisch gesichert.

Khellin besitzt den Vorteil, daß nach bisherigen Erkenntnissen keine phototoxischen Reaktionen auftreten und daß es nur ein geringes photomutagenes Potential besitzt - insbesondere im Vergleich mit 8-MOP. Einer der Gründe für das geringe photomutagene Potential kann die nur sehr geringe Fähigkeit von Khellin sein, Diaddukte auszubilden, die im Zusammenhang mit kanzerogenen Eingenschaften von Photosensibilisatoren gesehen werden. So wurde keine signifikante Mutagenität in Grünalgen und nur eine geringe Photomutagenität in Bakterien beobachtet. Tierversuche ergaben, daß eine UVA-Phototherapie mit Khellin ein deutlich geringeres karzinogenes Risiko besitzt als die bisher übliche Photochemotherapie mit 8-MOP. Weiterhin führt eine Khellin-UVA-Therapie nicht zu den bekannten, oben teilweise erwähnten, systemischen Nebenwirkungen der oralen Phototherapie mit 8-MOP (es ist keine Übelkeit beobachtet worden) und ist logistisch wesentlich einfacher und zeitsparender als die bisher bekannte Alternative, die Bade-PUVA-Therapie.

Die erfindungsgemäße Khellin-Zubereitung enthält Khellin und eine dermatologisch verträgliche Grundlage. Die kann in Form einer Salbe, einer Creme, eines Gels, einer Lotion, eines Sprays, eines Badezusatzes oder einer Paste vorliegen. Liegt sie als Creme vor, so beträgt die Menge an Khellin und seiner Derivate 0,1 - 15 Gew.-%, bevorzugt 5 Gew.-%. Je nach Psoralen kann die Konzentration bis auf 60-100 ng/mg Gewebe verdünnt werden.

Die Grundlage der Formulierung kann eine übliche ambiphile Creme- oder Salbengrundlage sein, z.B. Kohlenwasserstoffgrundlage wie Vaselin, Triglyceride, Wachse oder ein Polyalkylsiloxan.

Beispiele für eine Kohlenwasserstoffgrundlage sind Naturund Kunstvaseline, Plastibase und dick- und dünnflüssige Paraffine. Beispiele für eine Triglyceridgrundlage sind natürliche oder synthetische Triglyceridöle wie Oliven-, Erdnuß- und Ricinusöl oder Neutralöle.

Bevorzugt enthält die Zubereitung eine wässrige Phase und liegt in Form einer Creme, eines Gels, einer Lotion, eines Sprays, eines Badezusatzes oder einer Paste vor. Sie kann ein Ö/W-bzw. ein W/Ö-System sein. W/Ö-Absorptionsgrundlagen umfassen z.B. Fettalkohole wie Cetylalkohol und Stearylalkohol, Wollwachs und Wollwachsalkohole. Derartige Zubereitungen enthalten ferner in der Dermatologie übliche Tenside, Gerüstbildner, Emulgatoren, Farb-, Geruchs- und Konservierungsstoffe, sowie rezepturbedingte Formulierungshilfsmittel. Eine Mikroverkapselung des Wirkstoffs z.B. in Liposomen, Nanoparts, Nanosomen etc. ist ebenfalls möglich.

Typische Gelgrundstoffe sind z.B. Hydroxypropylcellulose, Methylhydroxyethylcellulose, Carboxymethylen, Propylenglykol, Ethanol 96%.

Ferner kann die erfindungsgemäße Zusammensetzung auch hautpflegende Zusätze enthalten wie z.B. Pflanzenöle (Mandelöl, Jojobaöl, Weizenkeimöl, Maiskeimöl, Erdnußöl etc.), Bienenwachs, Karitébutter, Sheabutter, Allantoin, Sojasterin, chamazulenreiche ätherische Öle, Harnstoff, Heilpflanzenauszüge wie Hamamelisblätterfluidextrakt, Auszug aus Kamillenblüten, Melissenblätter oder Ringelblumenblüten, hautverträgliche Vitamine oder Vitaminderivate wie Cholecalziferol, Dexpathenol, Retinolpalmitat oder Tocopherole, oder hautverträgliche Eiweißkonzentrate wie z.B. Sauermilchmolkekonzentrat.

Ferner kann die erfindungsgemäße Zusammensetzung noch weitere Wirkstoffe wie Vitamin A, Vitamin D, Vitamin E oder deren Derivate, Anthralin oder Kortikosteroide enthalten.

Die erfindgungsgemäße Khellin-Zubereitung eignet sich zur Verwendung bei der phototherapeutischen/ photochemotherapeutischen Behandlung aller Formen der Psoriasis, insbesondere der chronischen mittelschweren und schweren Form, einschließlich der Psoriasis palmoplantaris sowie der Hand- und Fußekzeme. Die Behandlung ist einfach durchzuführen. Eine normale Pigmentierung der Haut ist zu beobachten. Es traten aber keine phototoxischen Reaktionen oder ungewöhnliche Hyperpigmentierungen der Haut auf. Bei akzidenteller Sonneneinwirkung oder falscher Applikation wurden keine Verbrennungen beobachtet.

Die Menge der jeweils aufzubringenden Menge an Khellin hängt von dem Zustand des Patienten, der Schwere der Erkrankung, der Dauer der Behandlung etc. ab und wird vom behandelnden Arzt bestimmt. Üblicherweise beträgt die Dosis 1-10 J/cm² , die Behandlung wird etwa 4 mal pro Woche durchgeführt. Durchschnittlich werden 20-30 Behandlungen benötigt.

Die Erfindung wird anhand der nachstehenden Beispiele näher erläutert.

### Beschreibung der Behandlung:

Die Psoriasis-Plaques werden mit der Khellin-Zubereitung behandelt, die Khellin-Zubereitung wirkt eine Stunde ein. Anschließend wird mit UV-Strahlung behandelt. Anfangs werden niedrige Dosen verwendet, die im Laufe der Behandlung kontinuierlich gesteigert werden. Die Therapie wird initial 4-5x pro Woche durchgeführt, bei Abheilen der Läsionen wird eine Erhaltungstherapie 1x pro Woche für weitere 3-4 Behandlungen empfohlen.

### Beispiel 1

### Formulierung einer Khellin-Creme

Es wird eine Khellin-Creme (100g) hergestellt, die die folgenden Inhaltsstoffe enthält:

| | |
|---|---|
| Khellin | 5.0 |
| Basiscreme | ad 100.0 |

100 g der Basiscreme beinhalten Glycerolmonostearat 4.0, Cetylalkohol 6.0, mittelkettige Triglyceride 7.5, weißes Vaselin 25.5, Polyoxyethylenglycerolmonostearat 7.0, Propylenglycol 10.0 und Wasser 40.0.

Die vorstehenden Bestandteile wurden vermischt und in üblicher Weise zu einer Creme formuliert.

### Beispiel 2

### Mikroskopische Veränderungen unter der Khellin-UVA-Photochemotherapie

Sechs Patienten (3 Frauen, 3 Männer; durchschnittliches Alter 55 Jahre, 38-76 Jahre) litten seit über 10 Jahren unter Psoriasis. Bei den histologischen Untersuchungen waren eine Verdickung der Epidermis mit Verlängerung der Reteleisten und Munro'schen Mikroabszessen sichtbar. Im Korium wurden zahlreiche proentzündliche Lymphozyten und neutrophile Granulozyten festgestellt und damit die Diagnose einer Psoriasis bestätigt.

Es erfolgte eine Behandlung mit der erfindungsgemäßen Khellin-Creme in Kombination mit der UVA-Bestrahlung. Durchschnittlich erhielten die Patienten 23,2 Behandlungen. Nach der Behandlung mit der erfindungsgemäßen Khellin-UVA-Phototherapie zeigte sich eine weitgehende Normalisierung der gesamten Struktur der Epidermis bei allen Patienten mit Rückgang der proentzündlichen Lymphozyten. Diese Erfolge der Therapie sind vergleichbar mit Ergebnissen, die mit der konventionellen PUVA-Therapie mit 8-MOP erreicht werden können.

### Beispiel 3

### Vergleich der topischen Khellin-UVA-Therapie mit fest etablierten Phototherapien und Photochemotherapien anhand des anerkannten klinisch evaluierbaren "Psoriasis Activity and Severity Index" (PASI)-Scores

Die Wirksamkeit der Phototherapie unter Verwendung der erfindungsgemäßen Khellin-Creme wurde im Vergleich mit der Bade-PUVA- und der UVB-311nm-Phototherapie, zwei anerkannt wirksamen Therapieverfahren für Psoriasis, analysiert. Bei besonders schweren Formen der Psoriasis wurde die UVB-311-nm-Phototherapie in Kombination mit einer topischen Therapie wie Anthralin durchgeführt. Eine derartige Kombinationstherapie erfordert in der Regel eine stationäre Behandlung, da die Behandlung mit Anthralin zu Hause nicht durchgeführt werden kann.

Die Bade-PUVA-Therapie beruht auf dem Prinzip der Photosensibilisierung durch 8-MOP und anschließende UVA-Bestrahlung. Die UVB-311nm-Phototherapie nutzt im UVB-Anteil die Wellenlänge 311nm, da diese im Studienvergleich einer UVB-Breitspektrum-Behandlung überlegen war.

Dargestellt sind im Vergleich die "Psoriasis Activity and Severity Index" (PASI)-Scores. Dies ist ein Verlaufsparameter, der Schweregrad der Psoriasis und Therapieeffektivität im Verlauf klinisch dokumentiert.

| | topische Khellin Therapie | Bade-PUVA-Therapie | UVB-311 nm * |
|---|---|---|---|
| Gesamt n = | 32 | 40 | 144 |
| PASI-Therapie vor | 22,9 | 22,0 | 18,4 |
| PASI Woche 1 | 14,9 | 14,9 | 11,4 |
| PASI Woche 2 | 10,2 | 9,9 | 6,7 |
| PASI Woche 3 | 7,3 | 6,0 | 4,7 |
| PASI Woche 4 | 6,9 | 5,0 | 3,5 |
| PASI Woche 5 | 4,7 | 4,9 | 3,0 |

| | | | |
|---|---|---|---|
| * bei schwerer Psoriasis in Kombination mit Vit. D-Derivaten oder Anthralin) | | | |

Ein Vergleich der Creme-KUVA-Therapie, Patienten (n=32) mit Bade-PUVA (n=40) und UVB-311nm-Phototherapie (n=144) alleine oder in Kombination mit äußerlicher Therapie ist im Verlauf dargestellt. Ausgehend von einem mittleren PASI-Score von 22,9 vor Beginn der Therapie mit Creme-KUVA ist nach einer Woche (14,9), 3 Wochen (7,3), 4 Wochen (6,9) und 5 Wochen (4,7) eine rasche, deutliche und anhaltende Besserung des klinischen Zustandes eingetreten. Im Vergleich hierzu zeigen die Bade-PUVA-Therapie nach 5 Wochen (4,9) und die Therapie mit UVB-311nm-Bestrahlung ohne, oder mit äußerlicher Therapie (3,0) ähnliche gute Behandlungserfolge. Anzumerken ist, daß in der Creme-KUVA-Gruppe der Schweregrad der Psoriasis vor Therapiebeginn größer war (mittlerer PASI-Score 22,9), als in der mit UVB-311nm behandelten Gruppe (mittlerer PASI-Score 18,4). Zusammenfassend erweist sich die Creme-KUVA-Therapie als eine zumindest gleichschnelle und ebenso effiziente Behandlungform der Psoriasis wie die beiden etablierten Therapieformen.

### Beispiel 4

### Vergleich der Photochemotherapie bei Psoriasis palmoplantaris

Die Wirksamkeit der Phototherapie unter Verwendung der erfindungsgemäßen Khellin-Creme wurde im Vergleich mit der Bade-PUVA-Phototherapie bei der Psoriasis an Händen und Füssen, inklusive der pustulösen Formen, untersucht. Es zeigte sich, daß die Patienten bei der erfindungsgemäßen Khellin-Creme-Phototherapie durchschnittlich schneller ansprachen und insgesamt eine geringere Therapieanzahl benötigten. Die kumulative UVA-Dosis war durchschnittlich bei der erfindungsgemäßen Khellin-Creme-Phototherapie höher, bedingt durch die Möglichkeit einer schnelleren Steigerung aufgrund des Fehlens von phototoxischen Reaktionen bei der Therapie. Der Anteil an kompletten Remissionen, also vollständiger Abheilung, waren bei beiden Therapieverfahren vergleichbar.

| | topische Khellin-UVA-Therapie | Bade-PUVA-Therapie |
|---|---|---|
| Gesamtzahl der Patienten | 17 | 28 |
| weiblich/männlich | 8:9 | 12:16 |
| Alter | 54,1 | 51,0 |
| Therapieanzahl | 28,4 | 32,9 |
| Besserung nach Behandlungen | 7,9 | 10,4 |
| kumulative UVA-Dosis | 249,7 | 139,9 |
| komplette Remission | 7/17 | 12/29 |

### Beispiel 5

### Vergleich der Photochemotherapie bei Ekzemen

Die Wirksamkeit der Phototherapie unter Verwendung der erfindungsgemäßen Khellin-Creme wurde im Vergleich mit der Bade-PUVA-Phototherapie bei den chronischen Ekzemen, hier beispielhaft an Händen und Füssen untersucht. Es zeigte sich, daß die Patienten bei der erfindungsgemäßen Khellin-Creme-Phototherapie durchschnittlich weniger Therapien benötigten. Die kumulative UVA-Dosis war durchschnittlich bei der erfindungsgemäßen Khellin-Creme-Phototherapie höher, bedingt durch die schnellere Steigerung aufgrund des Fehlens von phototoxischen Reaktionen bei der Therapie. Der Anteil an kompletten Remissionen, also vollständiger Abheilung, waren bei beiden Therapieverfahren vergleichbar.

| | topische Khellin-UVA-Therapie | Bade-PUVA-Therapie |
|---|---|---|
| Gesamtzahl der Patienten | 11 | 19 |
| weiblich/männlich | 4:7 | 10:9 |
| Alter | 57,0 | 50,0 |
| Therapieanzahl | 21,5 | 26,1 |
| Besserung nach Behandlungen | 10.3 | 8,7 |
| kumulative UVA-Dosis | 181,0 | 83,0 |
| komplette Remission | 7/11 | 11/19 |

## Patentansprüche

1. Verwendung von Khellin zur Herstellung einer streichfähigen bzw. spreitfähigen, für die Applikation auf der Haut geeigneten Khellin-Zubereitung, enthaltend Khellin, sowie eine dermatologisch verträgliche Grundlage zusammen mit rezepturbedingten Hilfs- und Zusatzstoffen, als Photochemotherapie der Psoriasis, ihrer Subtypen und Sonderformen, einschließlich der Palmoplantar-Psoriasis, sowie als Photochemotherapie von Ekzemen, in einer Applikationsdosis von 0,1-15 Gew.-% bis zu einer Verdünnung auf 60-100 ng/mg Gewebe.

2. Verwendung von Khellin zur Herstellung eines Arzneimittels zur topischen UVA-Strahlenbehandlung der Psoriasis, ihrer Subtypen und Sonderformen, einschließlich der Palmoplantar-Psoriasis, sowie zur Behandlung von Ekzemen, in einer Applikationsdosis von 0,1-15 Gew.-% bis zu einer Verdünnung auf 60-100 ng/mg Gewebe.

3. Verwendung nach Anspruch 2 in Form einer Creme, einer Salbe, eines Gels, einer Lotio, eines Sprays, eines Badezusatzes oder einer Paste.

## Claims

1. Use of khellin in the manufacture of a khellin-preparation suitable for application to the skin by painting and/or spreading and containing khellin together with a dermatologically safe carrier, with excipients and additives as determined by the recipe, as a photo-chemotherapy for psoriasis, its sub-types and special forms, including palmoplantar psoriasis, and as a photo-chemotherapy for eczema, in an applied dosage of 0.1-15% by weight up to a dilution of 6-100 ng/mg tissue.

2. Use of khellin for the manufacture of a therapeutic agent for topical UVA-radiation treatment of psoriasis, its sub-types and special forms, including palmoplantar psoriasis, and for the treatment of eczemas, in an applied dosage of 0.1-15% by weight up to a dilution of 60-100 ng/mg tissue.

3. Use according to claim 2 in the fonn of a cream, an ointment, a gel, a lotion, a spray, a bath additive or a paste.

## Revendications

1. Emploi de khelline pour la fabrication d'une préparation étalable ou extensible de khelline, propre à être appliquée sur la peau et contenant de la khelline ainsi qu'une base dermatologiquement compatible et des adjuvants et auxiliaires de formulation, pouvant servir en photochimiothérapie du psoriasis, de ses sous-types et de ses formes particulières, y compris le psoriasis palmo-plantaire, ainsi qu'en photochimiothérapie de l'eczéma, et applicable en une concentration de 0,1 à 15 % en poids, jusqu'à une dilution à 60-100 ng par mg de tissu.

2. Emploi de khelline pour la fabrication d'un médicament destiné au traitement topique par rayons UV-A du psoriasis, de ses sous-types et de ses formes particulières, y compris le psoriasis palme-plantaire, ainsi qu'au traitement de l'eczéma, et applicable en une concentration de 0,1 à 15 % en poids, jusqu'à une dilution à 60-100 ng par mg de tissu.

3. Emploi selon la revendication 2, sous la forme d'une crème, d'une pommade, d'un gel, d'une lotion, d'un spray, d'un adjuvant de bain ou d'une pâte.
